Europäisches Patentamt

European Patent Office

Office européen des brevets

(19)

(11) Publication number : **0 394 837 B1**

# EUROPEAN PATENT SPECIFICATION

(12)

(45) Date of publication of patent specification :
07.07.93 Bulletin 93/27

(51) Int. Cl.[5] : **A23K 1/14,** A23K 1/16, A23K 1/18

(21) Application number : **90107426.0**

(22) Date of filing : **19.04.90**

(54) **Feed additive for cultivated fishes and feed for cultivated fishes.**

(30) Priority : **20.04.89 JP 101082/89**

(43) Date of publication of application :
**31.10.90 Bulletin 90/44**

(45) Publication of the grant of the patent :
**07.07.93 Bulletin 93/27**

(84) Designated Contracting States :
**DE ES FR GB IT**

(56) References cited :
DERWENT FILE SUPPLIER WPIL, 1989, acces-
sion no. 89-237712 [33], Derwent Publications
Ltd, London, GB; & JP-A-01 172 341 (NISSHIN
FLOUR MILL) 07-07-1989
DERWENT FILE SUPPLIER WPI, 1971, acces-
sion no. 71-63336S [39], Derwent Publications
Ltd, London, GB; & JP-B-71 034 176 (K.
IWABUCHI et al.) 1971

(56) References cited :
PATENT ABSTRACTS OF JAPAN, vol. 10, no.
310 (C-379)[2366], 22nd October 1986; & JP-
A-61 119 144 (OSAKA CHEM. LAB.) 06-06-1984
(Cat. D)
DERWENT FILE SUPPLIER WPIL, 1987, acces-
sion no. 87-034013 [05], Derwent Publications
Ltd, London, GB; & JP-A-61 291 525 (OSAKA
YAKUHIN KENKY) 22-12-1986

(73) Proprietor : **SAN-A PHARMACEUTICAL CO.,
LTD**
3-13-6, Saginomiya, Nakano-ku
Tokyo (JP)

(72) Inventor : **Kajitani, Shigeo**
15-2-902, Akabanekita 2-chome
Kita-ku, Tokyo (JP)

(74) Representative : **Laudien, Dieter, Dr.**
Boehringer Ingelheim Zentrale GmbH ZA
Patente Postfach 200
W-6507 Ingelheim am Rhein (DE)

EP 0 394 837 B1

## Description

### Background of the Invention:

The present invention relates to a feed additive for cultivated fishes such as yellowtails, sea breams, eels and sweetfishes.

Yellowtails are now cultivated on a large scale. In the course of their long period of cultivation, however, their health usually deteriorates owing to hepatic insufficiency brought on by the feed, a bad cultivation environment or stress caused by changes in water temperature of environment. Further the cultivated fishes are injured during transportation, changing of nets or sorting. Under these circumstances, feeds containing various additives for maintaining the fishes' health and promoting efficient cultivate them have been marketed. For example, feeds for cultivated fishes containing mugwort (Artemisia indica) powder, licorice (Glycyrrhiza glabra) powder or turmeric (Curcumae rhizoma) powder in order to maintain the cultivated fishes' health, to prevent deterioration of liver function and to prevent rancidity of live feeds are commercially available. Although the above-described effects are obtained when the feeds containing these additives are given to the fishes, the effects are still insufficient.

It has also been proposed to use a powder or extract of a plant selected from the group consisting of ginseng (Ginseng radix), watermelon, sweet hydrangea leaf, cucumber, melon, licorice, bupleurum (Bupleuri radix), adzuki beans and tea leaves in order to improve the quality of meats of cattle and cultivated fishes (Japanese Patent Unexamined Published Application (hereinafter referred to as 'J.P. KOKAI') No. 61-1 19144). However, no means for efficiently maintaining the cultivated fishes' health or for cultivating them has been developed yet.

### Summary of the Invention:

Therefore, a primary object of the present invention is to provide a feed additive for cultivated fishes capable of strengthening the internal organs of the fishes, to protect them from diseases and to efficiently increase their body weight. Another object of the present invention is to provide a feed containing the feed additive for cultivated fishes.

These and other objects of the present invention will be clear from the following description and Examples.

The present invention has been completed on the basis of a finding that when a feed containing a combination of at least two specific Chinese orthodox medicines (chinese herbal remedies) is given to cultivated fishes, diseases of the fishes can be quite effectively prevented and, therefore, the body weight of the fishes can be efficiently increased.

The present invention provides a feed additive for cultivated fishes characterized by comprising (a) weeping forsythia (Forsythiae fructus or Forsythiae suspensa Vahl) and (b) at least one of licorice (Glycyrrhizae radix or Glycyrrhiza glabra), bupleurum (Bupleuri radix) and cnidium rhizome (Cnidii rhizoma or Cnidium officinale Makino). The present invention provides also a feed for cultivated fishes containing such a feed additive.

### Description of the Preferred Embodiments:

The weeping forsythia (Forsythiae fructus) used as the indispensable ingredient (a) of the present invention is either a dried product or extract of Forsythia suspensa vahl which is a deciduous shrub of Oleaceae or fruits thereof. Further, forsythia fruit KOREA is also usable as the ingredient (a).

The licorice used as one of the indispensable ingredients (b) is either a dried product or extract of a leguminous plant containing glycyrrhizic acid or its salt as the main ingredient. The salt is preferably an alkali metal salt (including a partial salt) such as sodium, potassium or lithium salt thereof. Tripotassium salt thereof is particularly preferred. The licorice is preferably a dry extract thereof prepared by drying a licorice extract. The bupleurum is either a dry product or extract from a perennial plant of umbelliferae containing buplerum saponin as the main ingredient. The dry root thereof is particularly preferred. Cnidium rhizome is also a dry product or extract from a perennial plant of Umbelliferae containing cnidium lactone and sedanonic acid as the main ingredients. The dry root thereof is particularly preferred. Dry cnidium extract prepared by drying a soft extract obtained by extracting cnidium with water is preferred.

The present invention is characterized by using the combination of the ingredients (a) and (b). Although the ratio between the ingredients is not particularly limited, the weight ratio of the ingredient (a) to the ingredient (b) is usually 9/1 to 1/9, preferably 8/2 to 2/8. At least one of the ingredients (b) is used in combination with the ingredient (a). A further improved effect can be obtained when at least two of the ingredients (b) are used. In such a case, the weight ratio between the two ingredients (b) is preferably 9/1 to 1/9. A very excellent effect can be obtained when three ingredients (b) are used in combination with the ingredient (a) in the present invention. In this case, the weight ratio of licorice/bupleurum/cnidium is 1/(0.5 to 5)/(0.1 to 2).

The feed additive for cultivated fishes can be used in the form of a powdery mixture of the ingredients. Further it can be also in any form such as granules having any desired granular size prepared by using a granulating agent or binder.

The feed additive for cultivated fishes according to the present invention may further contain vitamins, minerals, antioxidants, antibiotics, antibacterial agents, etc.

The feed additive of the present invention can be added to an ordinary feed for cultivated fishes in an amount of 1 to 30 g (0.1 to 3%), preferably 3 to 15 g (0.3 to 1.5%) per kilogram of the feed. The feeds are ordinary ones such as live feeds, mixed feeds, pelletized feeds and moist pelletized feeds. In this connection, examples of live feeds include minced fish, cutted fish and whole fish such as sardine and anchovy. The mixted feeds are prepared by mixing fish meal as a main ingredient with vegitable oil residues, cereals and the like. The pelletized feeds comprise fish meal and wheat flour as main ingredients, while the moist pelletized feeds mean Oregon Moist Pellets which comprise about 50% by weight of the live feeds and about 50% by weigth of the mixed feeds.

The feeds for cultivated fishes can be easily produced by mixing the feed additive with an ordinary reed by an ordinary process.

The present invention thus provides a feed additive for cultivated fishes which comprises a combination of two kinds of chinese orthodox medicines and which is capable of quite effectively preventing diseases of the cultivated fishes by the synergism of the two ingredients and thereby efficiently increasing the body weight of the fishes, though the mechanism thereof has not yet been fully elucidated. Both components (a) and (b) are water-soluble, and they can be applied over a wide range.

The feeds containing the additive for cultivated fishes can be continuously given to young and adult cultivated fishes such as yellowtails, flatfishes, salmons, swellfishes, breams, trouts, carps, eels and sweetfishes.

The following non-limitative Examples will further illustrate the present invention.

Example 1

1% by weight of a mixture of weeping forsythia powder and licorice powder in a weight ratio of 1/2 was added to a feed to prepare the feed of the present invention for cultivated fishes. In this case, a dried extract of Forsythia suspensa vahl was used as the weeping forsythia powder while a dried extract containing tripotassium salt of glycyrrhizic acid was used as the licorice powder. The feed used was Oregon Moist Pellets. This feed was given to 17,000 young yellowtails for 90 days and the number of dead fishes was recorded.

As a control, the same feed but containing neither weeping forsythia powder nor licorice powder was used. It was given to 18,000 young yellowtails and the number of dead fishes was recorded in the same manner as above.

Only 15.4 fishes died per day on average when the feed additive of the present invention was given, while 17.0 fishes died per day on average in the control group. When the death index obtained by using the additive of the present invention is represented to be 100, that in the control group is as high as 110.3. It is apparent, therefore, that when the feed additive of the present invention for the cultivated fishes is used, the number of dead fishes can be effectively reduced.

Example 2

1% by weight of a mixture of weeping forsythia powder, licorice powder, bupleurum powder and cnidium rhizome powder in a weight ratio of 2/1/3/1 was added to the same feed as that of Example 1 to prepare the feed of the present invention for cultivated fishes. In this case, the weeping forsythia powder and the licorice powder were the same as those used in Example 1. A dry extract from a perennial plant of umbelliferae containing buplerum saponin as the main ingredient was used as the bupleurum powder and a dry extract from a perennial plant of Umbelliferae containing cnidium lactone and sedanonic acid as the main ingredients was used as the Cnidium rhizome poweder. The feed was given to 12,000 young yellowtails for 90 days and the number of dead fishes was recorded.

As a control, the same feed as above but free from the above-described four ingredients was used. It was given to 12,000 young yellowtails and the number of dead fishes was recorded in the same manner as above.

Only 8.4 fishes died per day on average when the feed additive of the present invention was given, while 10.0 fishes died per day on average in the control group. When the death index obtained by using the additive of the present invention is represented to be 100, that in the control group is as high as 119. It is apparent,

EP 0 394 837 B1

therefore, that when the feed additive of the present invention comprising the four ingredients is used, the number of dead fishes can be more effectively reduced.

Example 3

0.1 to 0.15% by weight of a mixture of weeping forsythia powder, licorice powder, bupleurum powder and cnidium powder in a weight ratio of 2/1/3/1 was added to Mixed Feed F-2 (a product of Fuji Seifun Co., Ltd. comprising 75% by weight of a fish meal, 22% by weight of $\alpha$-starch and 3% by weight of calcium phosphate, calcium carbonate, etc.) to prepare the feed of the present invention for cultivated fishes. In this case, the mixture was the same as that used in Example 2. The feed was given to eels for 50 days and the average number of dead eels per day was recorded. The number of the fishes at the start of the test and average number of deaths per day in the respective test groups are summarized in Table 1.

Table 1

| Test group | Number of cultivated eels | Average number of dead eels |
|---|---|---|
| 1 | 4,300 | 0.6 |
| 2 | 20,900 | 0.5 |
| 3 | 22,200 | 0.6 |
| 4 | 20,100 | 1.0 |
| 5 | 23,100 | 1.4 |
| 6 | 16,700 | 2.5 |
| Average | – | 1.1 |

The main cause for the death during the test period was Edwardsiellosis. Before the feed of the present invention for the cultivated eels was used in the test groups, 7 to 8 eels died a day on average. It is apparent from the results shown in Table 1 that when the feed of the present invention for cultivated fishes is used, the number of the dead eels can be efficiently reduced (the rate of survival can be increased).

**Claims**

1. A feed additive for cultivated fishes which comprises (a) weeping forsythia (Forsythiae fructus) and (b) at least one of licorice (Glycyrrhizae radix), bupleurum (Bupleuri radix) and cnidium rhizome (cnidii rhizoma).

2. A feed additive as set forth in claim 1 wherein the ingredient (a) is a dried product or extract of a deciduous shrub of Oleacese and fruits thereof.

3. A feed additive as set forth in claim 1 wherein the ingredient (b) is a dried product or extract of a leguminous plant containing glycyrrhizic acid or its salt.

4. A feed additive as set forth in claim 1 wherein the salt of glycyrrhizic is tripotassium salt thereof.

4

5. A feed additive as set forth in claim 1 wherein the ingredient (b) is a dried product or extract from a perennial plant of Umbelliferae containing buplerum saponin.

6. A feed additive as set forth in claim 1 wherein the ingredient (b) is a dried product or extract from a perennial plant of Umbelliferae containing cnidium lactone and sedanonic acid.

7. A feed additive as set forth in claim 1 wherein the weight ratio of the ingredient (a) to the ingredient (b) is 9/1 to 1/9.

8. A feed additive as set forth in claim 7 wherein two members of the ingredient (b) are used and the weight ratio of the two ingredients (b) is 9/1 to 1/9.

9. A feed additive as set forth in claim 7 wherein the three members of the ingredient (b) are used and the weight ratio of licorice/bupleurum/cnidium is 1/(0.5 to 5)/(0.1 to 2).

10. A feed for cultivated fishes which comprises an ordinary feed and the feed additive for cultivated fishes according to Claim 1.

11. A feed as set forth in claim 10 wherein the feed additive is added to an ordinary feed for cultivated fishes in an amount of 1 to 30 g per kg of the feed.

12. A feed as set forth in claim 10 wherein the ordinary feed is selected from the group consisting of live feeds, mixed feeds, pelletized feeds and moist pelletized feeds.


**Patentansprüche**

1. Futtermittelzusatz für Zuchtfische enhaltend (a) Forsythienfrüchte (Forsythiae fructus) und (b) wenigstens ein Bestandteil aus der Gruppe Süßholzwurzel (Glycyrrhizae radix), Wurzeln des Hasenohrs (Bupleuri radix) und den Rhizomen der Brenndolde (Cnidii rhizoma).

2. Futtermittelzusatz gemäß Anspruch 1, worin der Inhaltsstoff (a) durch ein Trockenprodukt oder einen Extrakt der jährlich abfallenden Blätter und der Früchte von pflanzen aus der Familie der Oleaceen verkörpert.

3. Futtermittelzusatz gemäß Anspruch 1, worin der Inhaltsstoff (b) ein Trockenprodukt oder einen Extrakt einer Hülsenfrüchte tragenden pflanze, enthaltend Glycyrrhizinsäure oder ein Salz derselben, verkörpert.

4. Futtermittelzusatz gemäß Anspruch 1, worin das Salz der Glycyrrhizinsäure durch deren Trikaliumsalz verkörpert wird.

5. Futtermittelzusatz gemäß Anspruch, worin der Inhaltsstoff (b) ein Trockenprodukt oder einen Extrakt einer mehrjährigen pflanze aus der Familie der Doldengewächse, enthaltend das Saponin des Bupleurums, verkörpert.

6. Futtermittelzusatz gemäß Anspruch 1, worin der Inhaltsstoff (b) ein Trockenprodukt oder Extrakt einer mehrjährigen pflanze aus der Familie der Doldengewächse, enthaltend das Lakton des Cnidiums und Sedanolsäure, verkörpert.

7. Futtermittelzusatz gemäß Anspruch 1, worin das Gewichtsverhältnis von Inhaltsstoff (a) zu Inhaltsstoff (b) 9/1 bis 1/9 beträgt.

8. Futtermittelzusatz gemäß Anspruch 7, worin die zwei Bestandteile der Inhaltsstoffe aus der Gruppe (b) in einem Gewichtsverhältnis von 9/1 bis 1/9 eingesetzt werden.

9. Futtermittelzusatz gemäß Anspruch 7, worin drei Bestandteile der Inhaltsstoffe aus der Gruppe (b) in einem Gewichtsverhältnis Süßholzwurzel/rundblättriges Hasenohr/Cnidium 1/ (0.5 bis 5 / (0.1 bis 2) beträgt.

10. Futtermittel für Zuchtfische enthaltend ein übliches Futtermittel und den Futtermittelzusatz für Zuchtfische gemäß Anspruch 1.

**11.** Futtermittel gemäß Anspruch 10, worin der Futtermittelzusatz in einer Menge von 1 bis 30 Gramm bezogen auf ein Kilogramm des eingesetzten Futtermittels dem üblichen Futtermittel zugesetzt worden ist.

**12.** Futtermittel gemäß Anspruch 10, worin das übliche Futtermittel aus der Gruppe, bestehend aus Lebendfuttermitteln, Mischfuttermitteln, pelletierten Futtermitteln und naß-pelletierten Futtermitteln, ausgewählt worden ist.

## Revendications

**1.** Additif alimentaire pour poissons d'élevage qui comprend (a) du forsythia pleureur (Forsythiae fructus) et (b) au moins l'un des éléments parmi la réglisse (Glycyrrhizae radix), le buplèvre (Bupleuri radix) et le rhizome de cnidium (cnidii rhizoma).

**2.** Additif alimentaire selon la revendication 1, dans lequel l'ingrédient (a) est un produit séché ou un extrait d'un arbuste à feuilles caduques de la famille des oléacées et de ses fruits.

**3.** Additif alimentaire selon la revendication 1, dans lequel l'ingrédient (b) est un produit séché ou un extrait d'une légumineuse contenant de l'acide glycyrrhizique ou l'un de ses sels.

**4.** Additif alimentaire selon la revendication 1, dans lequel le sel de l'acide glycyrrhizique est son sel tripotassique.

**5.** Additif alimentaire selon la revendication 1, dans lequel l'ingrédient (b) est un produit séché ou un extrait d'une plante pérenne de la famille des ombellifères contenant de la saponine de buplèvre.

**6.** Additif alimentaire selon la revendication 1 dans lequel l'ingrédient (b) est un produit séché ou un extrait d'une plante pérenne de la famille des ombellifères contenant de la lactone de cnidium et de l'acide sédanonique.

**7.** Additif alimentaire selon la revendication 1, dans lequel le rapport pondéral de l'ingrédient (a) à l'ingrédient (b) est de 9/1 à 1/9.

**8.** Additif alimentaire selon la revendication 7, dans lequel deux éléments de l'ingrédient (b) sont utilisés et le rapport pondéral des deux ingrédients (b) est de 9/1 à 1/9.

**9.** Additif alimentaire selon la revendication 7, dans lequel les trois éléments de l'ingrédient (b) sont utilisés et le rapport pondéral réglisse/buplèvre/cnidium est de 1/(0,5 à 5)/(0,1 à 2).

**10.** Aliment pour poissons d'élevage qui comprend un aliment ordinaire et l'additif alimentaire pour poissons d'élevage selon la revendication 1.

**11.** Aliment selon la revendication 10 dans lequel l'additif alimentaire est ajouté à un aliment pour poissons d'élevage ordinaire en une quantité de 1 à 30 g par kg d'aliment.

**12.** Aliment selon la revendication 10 dans lequel l'aliment ordinaire est choisi dans le groupe consistant en les aliments d'origine animale, les aliments mélangés, les aliments sous forme de boulettes et les aliments sous forme de boulettes humides.